# EUROPEAN PATENT APPLICATION

(11) **EP 4 393 433 A1**
(43) Date of publication of application: **03.07.2024**
(21) Application number: 23219837.4
(22) Date of filing: 22.12.2023
(51) Int. Cl.: A61B 18/14

(54) **BASKET CATHETERS WITH COMPATIBLE IRRIGATION AND STAGGERED ELECTRODES**

(30) Priority: 29.12.2022 US 202218091342
(71) Applicant: Biosense Webster (Israel) Ltd., 2066717 Yokneam (IL)
(72) Inventor: GOVARI, Assaf, 2066717 Yokneam (IL); ALTMANN, Andres Claudio, 2066717 Yokneam (IL); BEECKLER, Christopher Thomas, Irvine, 92618 (US); OKARSKI, Kevin Mark, Irvine, 92618 (US); KEYES, Joseph Thomas, Irvine, 92618 (US)
(74) Representative: Carpmaels & Ransford LLP

(57) **Abstract**

A medical probe includes a shaft, a basket assembly, a plurality of electrodes, and an irrigation source. The shaft is configured for insertion into an organ of a patient. The basket assembly is coupled at a distal end of the shaft, wherein the basket assembly includes a plurality of splines. The plurality of electrodes is fitted on a distal portion of the plurality of splines. The irrigation source is configured to deliver an irrigation fluid to the electrodes, wherein the irrigation source located proximally to the electrodes and distally to the distal end of the shaft.

## Description

### FIELD OF THE DISCLOSURE

The present disclosure relates generally to invasive medical probes, and particularly to cardiac catheters.

### BACKGROUND OF THE DISCLOSURE

Basket catheters typically have an elongated catheter body and a basket-shaped electrode assembly mounted at the distal end of the catheter body. The assembly has proximal and distal ends and comprises a plurality of splines connected at their proximal and distal ends. Each spline comprises at least one electrode that can be used for sensing and/or electrical ablation. The assembly has an axial elongated expander which is longitudinally movable relative to the catheter to vary the configuration of the basket between an expanded arrangement wherein the splines bow radially outwardly and a collapsed arrangement wherein the splines are arranged generally along the axis of the catheter body.

U.S. Patent Application Publication 2022/0071696, for example, describes a basket catheter assembly comprising a plurality of splines and a plurality of electrodes, each of the electrodes having a lumen therethrough fitting a given spline. The basket assembly has a distal edge and comprises a stem that extends longitudinally from a distal end of a shaft that carries the basket assembly the towards the distal edge. The stem comprises multiple spray ports, wherein each given spray port is angled to aim delivery of the irrigation fluid to a given electrode.

The present disclosure will be more fully understood from the following detailed description of the examples thereof, taken together with the drawings, in which:

### BRIEF DESCRIPTION OF THE DRAWINGS

Fig. 1 is a schematic, pictorial illustration of a catheter-based electrophysiology (EP) mapping and ablation system, in accordance with an example of the present disclosure;
Figs. 2A and 2B are schematic pictorial illustrations of an expandable assembly comprising an irrigation tube and splines carrying staggered electrodes, in accordance with examples of the present disclosure;
Fig. 3 is a schematic pictorial illustration of a staggered expandable assembly of Fig. 2 in a collapsed state, in accordance with examples of the present disclosure; and
Fig. 4 is a flow chart that schematically illustrates a method to apply a staggered basket catheter of Fig. 2 to treat a patient, in accordance with an example of the present disclosure.

### DETAILED DESCRIPTION OF EXAMPLES

### OVERVIEW

Basket-shaped catheters that can perform therapeutic electrical ablation typically include bulky electrodes (e.g., with a 1 mm diameter) capable of delivering a sufficient amount of ablative power. Such catheters further include an irrigation outlet from the distal end of the shaft (the stem) to cool the electrodes. However, irrigation from the distal end of the stem may not provide sufficient irrigation at the ablation site. Insufficient irrigation has been found to lead to blood coagulation on the electrodes during ablation. While locating the irrigation outlet closer to the distal end of the basket would be advantageous, this makes collapsing the basket to a small diameter for delivery via a sheath challenging.

Examples of the present disclosure that are described herein provide basket assembly designs with an irrigation source, such as comprising an extended irrigation tube on or near the center of the basket with the electrodes placed only at a distal portion (e.g., distal hemisphere) of the basket assembly. This division avoids contact between the irrigation tube and the electrodes in a collapsed state and therefore allows the basket to collapse to a small diameter.

In addition, the electrodes are arranged in a staggered fashion which also helps collapse the catheter to a small diameter. At least some of the electrodes may be arranged in pairs so that a physician can perform bipolar ablation in a concentrated area. Different options for electrode staggering in pairs are exemplified below.

### SYSTEM DESCRIPTION

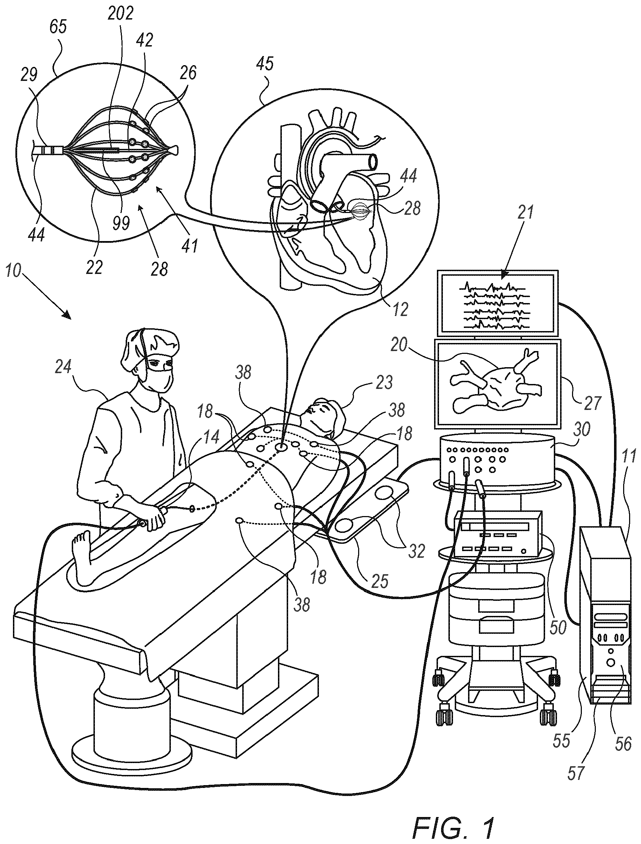

Fig. 1 is a schematic, pictorial illustration of a catheter-based electrophysiology (EP) mapping and ablation system 10, in accordance with an example of the present disclosure.

System 10 includes multiple catheters, which are percutaneously inserted by physician 24 through the patient's vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing intracardiac electrograms (IEGM) and/or for both sensing and ablating, as well as imaging catheters. An example basket catheter 14 that is configured for sensing IEGM and to perform electrical ablation is illustrated herein. As seen in inset 45, physician 24 brings a basket assembly 28 (also called hereinafter "expandable distal end assembly 28") fitted on a shaft 44 of catheter 14 into contact with the heart wall for sensing a target site in heart 12.

As seen in inset 65, basket catheter 14 is an exemplary catheter that includes one and preferably multiple electrodes 26 distributed in a staggered layout over a distal portion 41 of a plurality of splines 22 at expandable distal end assembly 28 and configured to sense the IEGM signals and perform ablation. Catheter 14 additionally includes (i) an expansion rod 42 to expand and collapse expandable assembly 28, (ii) a proximal position sensor 29 embedded in or near basket assembly 28 for tracking a position of a distal end of shaft 44, and (iii) an irrigation tube 202 to irrigate staggered electrodes 26 and/or tissue during ablation. As seen, irrigation tube 202 is comprised in a stem 99 that extends longitudinally from a distal end of shaft 44.

Magnetic-based position sensor 29 may be operated together with a location pad 25 including a plurality of magnetic coils 32 configured to generate magnetic fields in a predefined working volume. Details of the magnetic-based position-sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish location references for location pad 25 as well as impedance-based tracking functionality of electrodes 26. For impedance-based tracking, electrical current is directed toward electrodes 26 and sensed at electrode skin patches 38 so that the location of each electrode can be triangulated via the electrode patches 38. Details of the impedance-based location-tracking technology are described in US Patent Nos. 7,536,218; 7,756,576; 7,848,787; 7,869,865; and 8,456,182.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more electrodes at a distal tip of a catheter configured for ablation. Energy produced by ablation energy generator 50 may include, but is not limited to, radiofrequency (RF) energy or pulsed-field ablation (PFA) energy, including monopolar or bipolar high-voltage DC pulses as may be used to affect irreversible electroporation (IRE), or combinations thereof.

Patient interface unit (PIU) 30 is an interface configured to establish electrical communication between catheters, electrophysiological equipment, power supply and a workstation 55 for controlling system 10 operation. Electrophysiological equipment of system 10 may include, for example, multiple catheters, location pad 25, body surface ECG electrodes 18, electrode patches 38, ablation energy generator 50, and recorder 11. Optionally and preferably, PIU 30 additionally includes processing capability for implementing real-time computations of catheter locations and for performing ECG calculations.

Workstation 55 includes memory 57, processor 56 unit with memory or storage with appropriate operating software loaded therein, and user interface capability. Workstation 55 may provide multiple functions, optionally including (i) modeling the endocardial anatomy in three-dimensions (3D) and rendering the model or anatomical map 20 for display on a display device 27, (ii) displaying on display device 27 activation sequences (or other data) compiled from recorded electrograms 21 in representative visual indicia or imagery superimposed on the rendered anatomical map 20, (iii) displaying real-time location and orientation of multiple catheters within the heart chamber, and (iv) displaying on display device 27 sites of interest such as places where ablation energy has been applied. One commercial product embodying elements of system 10 is available as the CARTO^{™} 3 System, available from Biosense Webster, Inc., 31 Technology Drive, Suite 200, Irvine, CA 92618.

### BASKET CATHETERS WITH COMPATIBLE IRRIGATION AND STAGGERED ELECTRODES

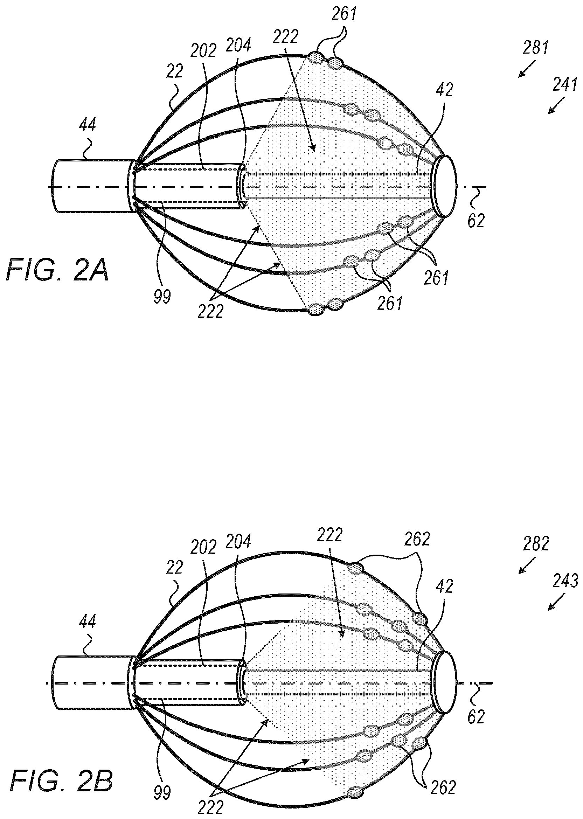

Figs. 2A and 2B are schematic pictorial illustrations of an expandable assembly (281, 282, respectively) comprising an irrigation tube and splines carrying staggered electrodes (261, 262, respectively), in accordance with examples of the present disclosure.

As seen in the figures, expandable assemblies 281 and 282 are coupled to a distal end of shaft 44, with expandable assemblies 281 and 282 extending along a longitudinal axis 62 and comprising a plurality of expandable splines 22 disposed about longitudinal axis 62 to define an internal lumen, such as one defined by a surface of revolution about longitudinal axis 62. Assemblies 281 and 282 can be, by way of example, self-expanding. In such case, extension rod 42 is not required, assembly can be withdrawn into sheath at the end of a procedure. The withdrawal process collapses the assembly sufficiently to fit within the sheath. When expanded, assemblies 281 or 282 configured to access tissue with electrodes 261 or 262 in confined cardiac regions such as that of an ostium of a pulmonary vein.

In the configuration shown in Fig. 2, pairs of electrodes 261 and/or 262 are fitted over splines 22. Electrodes 261 and 262 can be configured to deliver ablation energy to tissue in heart 12. In addition to using electrodes 261 and 262 to deliver ablation energy, the electrodes can also be used to determine the location of basket assemblies 281 and 282 and/or to measure an electrophysiological property such as local surface electrical potentials at respective locations on tissue in heart 12.

Electrodes 261 and 262 have sufficient surface area to be capable of delivering a sufficient amount of ablative power. Since electrodes 261 and 262 do not comprise spray ports to deliver irrigation fluid 222, the configuration described hereinabove enables heat to be transferred from the tissue (i.e., during an ablation procedure) to the portion of the electrodes on the inner side of the splines. The electrodes can be cooled by aiming irrigation fluid 222 via an irrigation source (202, 204), such as a spray sleeve 204 at the edge of irrigation tube 202. As seen, irrigation tube 202 is comprised in stem 99 that extends longitudinally from a distal end of shaft 44.

In other examples, multiple tubules can be used instead of a sleeve, or in a combined design with the sleeve. Using tubules or changing the sleeve profile along its circumference can be done to enhance flow of irrigation at particular directions, where the splines are oriented.

In both examples of Fig. 2A and 2B, the electrodes are located on the distal portion (e.g., respective distal hemispheres 241 and 243) of basket assemblies 281 and 282. This prevents contact between the irrigation tube and the electrodes in a collapsed state and therefore allows the basket to collapse to a small diameter.

In addition, the electrodes are arranged in a staggered fashion. This also helps collapse the catheter to a small diameter. Different options for staggering the electrodes (261, 262) in pairs are exemplified in Figs. 2A and 2B of assemblies (281, 282), respectively.

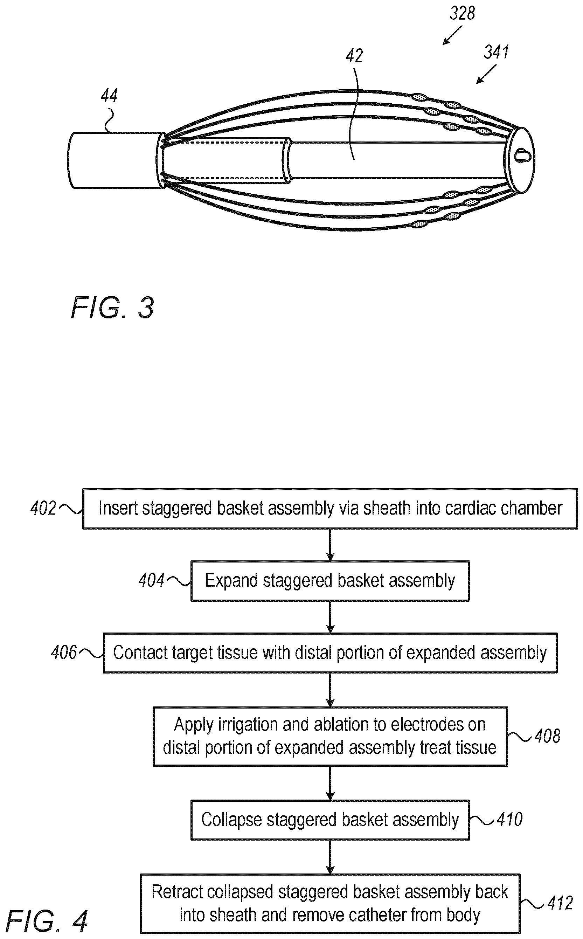

Fig. 3 is a schematic pictorial illustration of a staggered expandable assembly of Fig. 2 in a collapsed state, in accordance with examples of the present disclosure. As seen, the electrodes accommodate one another (e.g., do not overlap), and additionally accommodate (e.g., do not overlap with) the irrigation tube to allow the basket to collapse to a small diameter. In this way a lower diameter sheath may be used, which gives better access to organs of the human body.

### A METHOD TO APPLY A BASKET CATHETER OF FIG. 2 TO TREAT A PATIENT

Fig. 4 is a flow chart that schematically illustrates a method to apply a staggered basket catheter of Fig. 2 to treat a patient, in accordance with an example of the present disclosure. The algorithm, according to the presented example, carries out a process that begins with physician 24 inserting staggered basket assembly 28 via a sheath into a cardiac chamber of a patient, at catheter insertion step 402.

Next, the physician advances the catheter distally to the sheath and expands the staggered basket assembly, at a basket expansion step 404.

At catheter positioning step 406, the physician manipulates the catheter to bring the staggered electrodes mounted on the distal portion of the basket assembly into firm contact with tissue, so as to ablate target tissue (e.g., an ostium of a pulmonary vein).

The physician then applies irrigation and ablative power to the staggered electrodes, at a catheter ablation step 408.

Once ablation is completed, the physician withdraws and collapses the basket assembly, at basket collapsing step 410. Thanks to the staggered electrodes accommodating one another, as well as accommodating the irrigation tube, the basket can be collapsed to a small diameter.

Finally, the physician retracts the collapsed basket assembly back into the sheath and removes the catheter from the patient's body, at a catheter removal step 412.

The flow chart shown in Fig. 4 is chosen purely for the sake of conceptual clarity. The present example may also comprise additional steps of the algorithm, such as estimating contact force of electrodes against tissue. This and other possible steps are omitted from the disclosure herein purposely in order to provide a more simplified flow chart.

### EXAMPLES

### Example 1

A medical probe (14) includes a shaft (44), a basket assembly (281, 282), a plurality of electrodes (261, 262), and an irrigation source (202, 204). The shaft is configured for insertion into an organ of a patient. The basket assembly is coupled at a distal end of the shaft, wherein the basket assembly includes a plurality of splines (22). The plurality of electrodes is fitted on a distal portion (241, 243) of the plurality of splines. The irrigation source is configured to deliver an irrigation fluid (222) to the electrodes, wherein the irrigation source located proximally to the electrodes and distally to the distal end of the shaft.

### Example 2

The medical probe according to example 1, wherein the electrodes (261, 262) and the irrigation source (202, 204) do not overlap one another when the basket assembly (281, 282) is in a collapsed layout.

### Example 3

The medical probe according to any of examples 1 and 2, wherein at least some of the electrodes (261, 262) are arranged in pairs over respective splines (22) to enable bi-polar ablation.

### Example 4

The medical probe according to any of examples 1 through 3, wherein the electrodes (261, 262) are arranged staggered between splines (22) so that the electrodes do not overlap one another when the basket assembly (281, 282) is in a collapsed layout.

### Example 5

The medical probe according to any of examples 1 through 4, wherein the irrigation source (202, 204) comprises an irrigation tube (202) configured to irrigate the electrodes via a sleeved outlet (204).

### Example 6

The medical probe according to any of examples 1 through 5, wherein the irrigation source (202, 204) comprises an irrigation tube (202) configured to irrigate the electrodes via a set of spray ports.

### Example 7

A medical method includes inserting a shaft (44) having a basket assembly (281, 282) at a distal end of the shaft into an organ of a patient, the basket assembly comprising a plurality of splines (22) and a plurality of electrodes (261, 262) fitted on a distal portion (241, 243) of the plurality of splines. An irrigation fluid (222) is delivered to the electrodes using an irrigation source located proximally to the electrodes and distally to the distal end of the shaft.

Although the examples described herein mainly address cardiac diagnostic applications, the methods and systems described herein can also be used in other medical applications.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

## Claims

1. A medical probe (14), comprising:
a shaft (44) for insertion into an organ of a patient;
a basket assembly (281, 282) at a distal end of the shaft, the basket assembly comprising a plurality of splines (22);
a plurality of electrodes (261, 262) fitted on a distal portion (241, 243) of the plurality of splines; and
an irrigation source (202, 204) configured to deliver an irrigation fluid (222) to the electrodes, the irrigation source located proximally to the electrodes and distally to the distal end of the shaft.

2. The medical probe according to claim 1, wherein the electrodes (261, 262) and the irrigation source (202, 204) do not overlap one another when the basket assembly (281, 282) is in a collapsed layout.

3. The medical probe according to claim 1 or claim 2, wherein at least some of the electrodes (261, 262) are arranged in pairs over respective splines (22) to enable bi-polar ablation.

4. The medical probe according to any preceding claim, wherein the electrodes (261, 262) are arranged staggered between splines (22) so that the electrodes do not overlap one another when the basket assembly (281, 282) is in a collapsed layout.

5. The medical probe according to any preceding claim, wherein the irrigation source (202, 204) comprises an irrigation tube (202) configured to irrigate the electrodes via a sleeved outlet (204).

6. The medical probe according to any preceding claim, wherein the irrigation source (202, 204) comprises an irrigation tube (202) configured to irrigate the electrodes via a set of spray ports.

7. A medical method, comprising:
inserting a shaft (44) having a basket assembly (281, 282) at a distal end of the shaft into an organ of a patient, the basket assembly comprising a plurality of splines (22) and a plurality of electrodes (261, 262) fitted on a distal portion (241, 243) of the plurality of splines; and
delivering an irrigation fluid (222) to the electrodes using an irrigation source located proximally to the electrodes and distally to the distal end of the shaft.

8. The medical method according to claim 7, wherein the electrodes (261, 262) and the irrigation source (202, 204) do not overlap one another when the basket assembly is in a collapsed layout.

9. The medical method according to claim 8, wherein at least some of the electrodes are arranged in pairs over respective splines (22) to enable bi-polar ablation.

10. The medical method according to claim 6, wherein the electrodes (261, 262) are arranged staggered between splines (22) so that the electrodes do not overlap one another when the basket assembly is in a collapsed layout.

11. The medical method according to claim 6, wherein the irrigation source (202, 204) comprises an irrigation tube (202) configured to irrigate the electrodes via a sleeved outlet.

12. The medical method according to claim 6, wherein the irrigation source (202, 204) comprises an irrigation tube (202) configured to irrigate the electrodes via a set of spray ports.
